# FASCICULE DE BREVET EUROPEEN

(11) **EP 1 176 190 B1**
(45) Date de publication et mention de la délivrance du brevet: **05.12.2012**
(21) Numéro de dépôt: 01401958.2
(22) Date de dépôt: 20.07.2001
(51) Int. Cl.: C12N 5/07

(54) **Milieux de culture cellulaire, notamment pour la culture de follicules ou d'embryons.**
Zellkulturmedium insbesondere für Kultur von Follikel oder Embryonen
Cell culture medium, specially for culture of follicles or embryos

(30) Priorité: 24.07.2000 FR 0009694
(43) Date de publication de la demande: 30.01.2002
(73) Titulaire: LABORATOIRE C.C.D., 75008 Paris (FR)
(72) Inventeur: Choay, Patrick, F-75007 Paris (FR); Weinman, Serge, F-75013 Paris (FR)
(74) Mandataire: Grosset-Fournier, Chantal Catherine

(56) Documents cités:
- WO-A-96/12793
- LOO D T ET AL: "Differentiation of serum-free mouse embryo cells into astrocytes is accompanied by induction of glutamine synthetase activity." JOURNAL OF NEUROSCIENCE RESEARCH, vol. 42, no. 2, 1995, pages 184-191, XP000993518 ISSN: 0360-4012
- ROY SHYAMAL K: "Epidermal growth factor and transforming growth factor-beta modulation of follicle-stimulating hormone-induced deoxyribonucleic acid synthesis in hamster preantral and early antral follicles." BIOLOGY OF REPRODUCTION, vol. 48, no. 3, 1993, pages 553-557, XP000993507 ISSN: 0006-3363

## Description

La présente invention a pour objet des compositions utilisables en tant que milieux de culture cellulaire, notamment pour follicules en cours de développement ou pour le développement d'embryons, et plus particulièrement d'embryons de mammifères, notamment d'embryons humains, éventuellement congelés.

Après maturation du follicule de De Graaf, l'ovulation permet à l'ovocyte de passer dans la trompe de Fallope pour y être éventuellement fécondé. L'embryon qui en résulte y demeure 3 à 5 jours pour se développer et atteindre les stades morula, puis blastocyste.

Dans la trompe, les gamètes, l'embryon, la morula, puis le blastocyste sont environnés par le fluide tubaire. La composition de ce dernier est complexe : elle associe les composants fondamentaux du milieu intérieur de la mère à ceux du liquide folliculaire, et elle ne semble pas subir d'importants changements qualitatifs au cours des premiers jours de la phase post-ovulatoire. Parvenu au stade blastocyste, l'embryon passe dans l'utérus dont la muqueuse a alors atteint un état de développement favorable pour l'implantation et la nidation de l'oeuf.

Le passage de l'embryon dans l'utérus ne s'effectue que lorsque l'un et l'autre sont parvenus au niveau de développement physiologique compatible avec l'implantation et la nidation : stade blastocyste pour l'embryon, endomètre proche de sa phase sécrétoire pour l'utérus.

Dans le cas de l'assistance médicale à la procréation, ces conditions physiologiques suggèrent un milieu de culture unique et constant, et non pas séquentiel comme il en existe actuellement, associant tous les éléments nécessaires au développement, de la fécondation *in vitro,* éventuellement consécutive à l'ICSI (*intra cytoplasmic sperm injection,* ou injection intracytoplasmique de spermatozoïdes), jusqu'au transfert de l'embryon.

La présente invention découle de la mise en évidence par les Inventeurs de compositions constantes utilisables aussi bien pour la culture de follicules que pour le développement de l'embryon jusqu'au stade blastocyste, et ce avec des rendements comparables, voire supérieurs, à ceux obtenus avec les compositions actuellement sur le marché.

Le rôle des facteurs de croissance (encore désignés GF, *growth factors)* sur le développement *in vitro* des follicules est bien connu chez le mammifère. Roy [Roy Shyamal K (1993), Biology of Reproduction, 48(3) : 553-557] décrit des compositions pour la culture *in vitro* de follicules comprenant au moins deux facteurs de croissance, lesquels ne sont jamais associés à un composé de la famille des corticoïdes.

La présente invention a pour objet des compositions pour la culture *in vitro* de follicules en cours de développement en vue de la maturation des ovocytes contenus dans lesdits follicules ou pour le développement d'embryons, éventuellement après décongélation des follicules ou des embryons, caractérisées en ce qu'elles comprennent au moins deux facteurs de croissance en association avec au moins un composé de la famille des corticoïdes impliqués dans la production énergétique chez les mammifères, et, le cas échéant, avec au moins un coenzyme clé du métabolisme énergétique, tel que le NAD/NADH et le NADP/NADPH, lesdits facteurs de croissance étant choisis parmi :
- le facteur de croissance hépatique, encore désigné HGF *(hepatocyte growth factor*),
- le facteur de croissance de transformation α, encore désigné TGFα (*transforming growth factor*),
- le facteur de stimulation de colonies de granulocytes et de macrophages, encore désigné GM-CSF (*granulocyte-macrophage colony stimulating factor*),
- le facteur épidermique de croissance, encore désigné EGF (epidermal growth factor) et/ou HB-EGF (*heparin-binding epidermal growth factor*),
- les facteurs de croissance et de différenciation, encore désignés GDF (*growth differenciation factors*), tels que le GDF-9,
- les facteurs de croissance semblables-à-l'insuline, encore désignés IGF *(insuline-like growth factors*), tels que l'IGF-1 et/ou l'IGF-2.

Avantageusement, les facteurs de croissance contenus dans les compositions de l'invention appartiennent à 5 familles de cytokines. Les hormones et les facteurs de croissance sont présents à des concentrations équilibrées, compatibles avec les conditions physiologiques. Les compositions de l'invention apportent aux cellules en croissance et en différenciation les molécules régulatrices dont elles ont impérativement besoin. Ainsi, ces compositions sont susceptibles de permettre la maturation des ovocytes humains immatures cultivés en présence de cellules folliculaires. Elles peuvent aussi assurer le développement de l'embryon humain jusqu'au stade blastocyste. Or, ce stade a un plus fort pourcentage d'implantation dans l'utérus que lorsque le transfert de l'embryon a lieu à un stade moins avancé, notamment aux stades de deux à environ huit cellules.

Avantageusement, les compositions de l'invention contiennent au moins trois facteurs de croissance choisis parmi ceux susmentionnés.

Avantageusement, les compositions de l'invention contiennent des facteurs de croissance humains recombinants.

Des compositions particulièrement préférées contiennent au moins de l'IGF-1 et/ou de l'IGF-2.

De préférence encore, les compositions susmentionnées de l'invention comprennent tous les facteurs de croissance susmentionnés.

De préférence, les concentrations en facteurs de croissance et de différenciation dans les compositions susmentionnées de l'invention sont nanomolaires, et avantageusement comprises entre environ 0,25 µg/L et environ 60 µg/L, notamment entre environ 0,5 µg/L et environ 50 µg/L.

Avantageusement les concentrations des facteurs de croissance et de différenciation contenus dans les compositions de l'invention sont telles que :
- la concentration d'EGF est d'environ 40 µg/L à environ 60 µg/L, notamment d'environ 50 µg/L,
- la concentration de TGFα est d'environ 20 µg/L à environ 30 µg/L, notamment d'environ 25 µg/L,
- la concentration d'HGF est d'environ 40 µg/L à environ 60 µg/L, notamment d'environ 50 µg/L,
- la concentration de GM-CSF est d'environ 1,25 µg/L à environ 1,75 µg/L, notamment d'environ 1,5 µg/L,
- la concentration de GDF-9 est d'environ 4 µg/L à environ 6 µg/L, notamment d'environ 5 µg/L,
- la concentration de IGF-1 est d'environ 12,5 µg/L à environ 17,5 µg/L, notamment d'environ 15 µg/L,
- la concentration de IGF-2 est d'environ 12,5 µg/L à environ 17,5 µg/L, notamment d'environ 15 µg/L.

L'invention a également pour objet les compositions susmentionnées comprenant un composé de la famille des corticoïdes impliqués dans la production énergétique chez les mammifères, et plus particulièrement de la famille des glucocorticoïdes, tel que l'hydrocortisone ou un dérivé de la même famille, de préférence sous une forme soluble en milieu aqueux.

Avantageusement, l'hydrocortisone susmentionnée se présente sous forme de sel hydrosoluble, notamment sous forme d'hémisuccinate d'hydrocortisone. De préférence, la concentration du sel d'hydrocortisone dans les compositions susmentionnées, est comprise entre environ 5 x 10⁻⁷ M et environ 10⁻⁶ M, et est notamment d'environ 7 x 10⁻⁷ M, soit environ 350 µg/L pour l'hémisuccinate d'hydrocortisone.

Avantageusement, les compositions définies ci-dessus contiennent des coenzymes clé du métabolisme énergétique susmentionnés, notamment à des concentrations telles que :
- la concentration de NAD/NADH soit d'environ 1 mg/L,
- la concentration de NADP/NADPH soit d'environ 1 mg/L.

L'invention concerne également les compositions définies ci-dessus se présentant sous forme de lyophilisat, à savoir sous forme de compositions dont les différents constituants sont amenés à l'état sec, et sont susceptibles d'être remis en solution en gardant leurs propriétés physico-chimiques et biologiques.

A ce titre, l'invention a plus particulièrement pour objet les lyophilisats susmentionnés, contenant :
- au moins deux facteurs de croissance tels que définis ci-dessus, ou la totalité de ces facteurs,
- au moins un composé de la famille des corticoïdes défini ci-dessus, tel que l'hémisuccinate d'hydrocortisone,
- et, le cas échéant, au moins un coenzyme clé du métabolisme énergétique défini ci-dessus, tel que le NAD/NADH et le NADP/NADPH.

L'invention a également pour objet l'application des compositions ou lyophilisats susmentionnés, en tant qu'adjuvants effecteurs pour la préparation de milieux de culture *in vitro* de follicules en cours de développement en vue de la maturation des ovocytes contenus dans lesdits follicules ou pour la culture d'embryons, éventuellement après décongélation des follicules ou des embryons, lesdits milieux de culture *in vitro* comprenant une composition telle que définie ci-dessus, en association avec les éléments utilisés classiquement dans le cadre de la culture des follicules ou des embryons, lesdits éléments étant choisis notamment parmi la sérum albumine humaine, ou bovine, le cas échéant recombinantes, et/ou les sels minéraux, et/ou les molécules énergétiques telles que le glucose, le pyruvate, et le lactate, et/ou les aminoacides pour la biosynthèse des protéines, et/ou les bases puriques et pyrimidiques pour la biosynthèse des acides nucléiques, et/ou des phospholipides ou du cholestérol pour la formation des membranes cellulaires, et/ou des vitamines, telles que des vitamines du groupe B, notamment la vitamine B5 (pantothènate), la vitamine B8 (biotine), la vitamine B1 (thiamine) et/ou de la vitamine C.

L'invention concerne également un procédé de préparation de milieux de culture *in vitro* définis ci-dessus, caractérisé en ce qu'il comprend une étape de mélange d'une composition telle que définie ci-dessus, le cas échéant après dissolution dans un volume approprié d'une composition susmentionnée sous forme de lyophilisat, avec une solution contenant les éléments utilisés classiquement dans le cadre de la culture des follicules ou des embryons, lesdits éléments étant tels que définis ci-dessus.

L'invention a plus particulièrement pour objet les milieux de culture *in vitro* de follicules en cours de développement en vue de la maturation des ovocytes contenus dans lesdits follicules ou pour la culture d'embryons, éventuellement après décongélation des follicules ou des embryons, lesdits milieux étant tels qu'obtenus par mise en oeuvre d'un procédé susmentionné, et étant caractérisés en ce qu'ils comprennent une composition telle que définie ci-dessus, en association avec les éléments susmentionnés utilisés classiquement dans le cadre de la culture des follicules ou des embryons.

L'invention a également pour objet un procédé *in vitro* de maturation des follicules de mammifères en cours de développement en vue de la maturation des ovocytes contenus dans lesdits follicules, et plus particulièrement de follicules humains, le cas échéant après décongélation de follicules préalablement congelés, caractérisé en ce qu'il comprend une étape de mise en culture *in vitro* de follicules prélevés chez la femelle, et plus particulièrement chez la femme, dans un milieu de culture tel que défini ci-dessus selon l'invention, avantageusement pendant environ 3 jours à environ 6 jours.

Avantageusement, les compositions de l'invention peuvent être utilisées à la fois pour la maturation des ovocytes et la maturation des embryons, notamment jusqu'au stade de blastocyste, éventuellement après décongélation des follicules ou des embryons.

L'invention a également pour objet un kit (ou trousse) pour la préparation extemporanée d'un milieu de culture tel que défini ci-dessus selon l'invention, notamment dans le cadre de la mise en oeuvre d'un procédé susmentionné, ce kit comprenant :
- une composition sous forme de lyophilisat, telle que décrite ci-dessus,
- et, le cas échéant, une solution aqueuse contenant les éléments utilisés classiquement dans le cadre de la culture des follicules ou des embryons, tels que les éléments définis ci-dessus, notamment un milieu commercial tel que le milieu Upgraded B9 de CCD.

La présente invention sera davantage illustrée à l'aide de la description détaillée qui suit d'exemples de milieux de culture selon l'invention.

A titre de solution aqueuse telle que définie ci-dessus, le milieu de culture Upgraded B9 CCD disponible commercialement comprend principalement les composés suivants :
- des sels minéraux : KCl, NaCl, MgSO₄, NaHCO₃, Na₂HPO₄, KH₂PO₄,
- les acides aminés essentiels, ainsi que d'autres acides aminés tels que l'acide glutamique, la glycine, la taurine, la cystéine et la glutamine,
- des oses et dérivés métaboliques, tels que glucose, pyruvate, lactate, acétate,
- des vitamines, notamment des vitamines du groupe B et de la vitamine C,
- des bases puriques et pyrimidiques,
- des antibiotiques : pénicilline G, streptomycine.

### Formule n°1

Dans cette formule, le milieu de culture Upgraded B9 CCD correspondant à la solution aqueuse définie ci-dessus, éventuellement additionné d'insuline humaine (notamment d'insuline recombinante à raison de 5 mg/L, sur la base de 1 mg = 25 U), est complété par le lyophilisat contenant :
- de l'hémisuccinate d'hydrocortisone (7 x 10⁻⁷ M, soit 350 µg/L),
- des facteurs de croissance humains recombinants : EGF (50 µg/L), TGFα (25 µg/L), HGF (50 µg/L), GM-CSF (1,25 µg/L), GDF-9 (5 µg/L),
- du NAD/NADH (1mg/L) et du NADP/NADPH (1mg/L).

### Formule n°2

Dans cette formule, le milieu de culture Upgraded B9 CCD, contenant éventuellement de l'insuline humaine, est complété par le lyophilisat contenant :
- de l'hémisuccinate d'hydrocortisone (7 x 10⁻⁷ M, soit 350 µg/L),
- des facteurs de croissance humains recombinants : EGF (50 µg/L), TGFα (25 µg/L), HGF (50 µg/L), GM-CSF (1,25 µg/L), GDF-9 (5 µg/L),
- IGF-1 (12,5 µg/L), IGF-2 (12,5 µg/L),
- et du NAD/NADH (1mg/L) et du NADP/NADPH (1mg/L).

### A) PREPARATION D'UN MILIEU DE CULTURE POUR LES APPLICATIONS SUSMENTIONNEES SELON LA FORMULE N°1

Le milieu de culture doit être préparé extemporanément par mélange de deux flacons :
- un flacon A, qui se présente sous la forme d'une solution d'un volume de 10 ml et qui contient le milieu Upgraded B9 dans lequel est éventuellement dissoute l'insuline;
- un flacon B, qui se présente sous la forme d'un lyophilisat et qui contient l'hémisuccinate d'hydrocortisone et les facteurs de croissance, le NAD/NADH et le NADP/NADPH.

Au moment de l'emploi, reprendre le lyophilisat du flacon B par un faible volume (~ 0,5 ml) de la solution du flacon A. Transférer le contenu du flacon B ainsi dissous dans le flacon A. Homogénéiser. Cette opération dilue donc au dixième la concentration initiale des constituants du flacon B.

### Fabrication du flacon A

Dans le cas de l'addition d'insuline, il convient de préciser qu'à l'état cristallin, les molécules d'insuline sont unies entre elles par des forces intermoléculaires qui ne se rompent qu'en milieu acide. Pour être mise en solution aqueuse, l'insuline doit donc être dissoute à pH 3 (HCI 0,1 N). Le pH de la solution est ensuite amené à 7,5 (NaOH 0,1N); cette opération doit être effectuée le plus rapidement possible, afin d'éviter des altérations de la molécule. Un léger précipité peut apparaître à pH5 ; il se redissout ensuite.

Préparer une solution à 100 U/ml. Ajouter la solution d'insuline au milieu Upgraded B9, dans les proportions de 1,25 ml pour 1 L, respectivement. Répartir en flacons de 10 ml qui seront fermés. Conserver la solution à +4°C. Le bulletin analytique de l'insuline, comportant, entre autres, l'activité et, éventuellement, les tests de sécurité, doit être joint ; celui-ci doit être communiqué par le fournisseur de l'insuline.

### Fabrication du flacon B

Une solution aqueuse préparée extemporanément contenant l'hémisuccinate d'hydrocortisone et les facteurs de croissance est répartie dans des flacons de 1 ml, de telle façon que chaque flacon contienne 3,5 µg d'hémisuccinate d'hydrocortisone, 500 ng d'EGF, 250 ng de TGFα, 500 ng d'HGF, 12,5 ng de GM-CSF, 50 ng de GDF-9, 10 µg de NAD/NADH et 10 µg de NADP/NADPH. Les flacons sont immédiatement lyophilisés. Conserver le lyophilisat à + 4°C.

### Conditions de fabrication

Toutes les opérations sont effectuées avec des matières premières stériles et en milieu stérile.

### B) PREPARATION D'UN MILIEU DE CULTURE POUR LES APPLICATIONS SUSMENTIONNEES SELON LA FORMULE N°2

IGF-1 et IGF-2 sont ajoutés à la solution initiale contenant les autres constituants du flacon B, pour obtenir 125 ng de chacun par flacon, puis l'ensemble est lyophilisé.

Les conditions de fabrication et de conservation sont les mêmes que celles du milieu préparé selon la formule n°1.

### C) PREPARATION D'UN MILIEU CONTENANT DE L'HYDROCORTISONE ET DES FACTEURS DE CROISSANCE

Dans une première étape, un lot expérimental de milieu contenant le glucocorticoïde et des facteurs de croissance a été préparé. Ce milieu de culture était constitué de milieu Upgraded B9 complété seulement par de l'insuline humaine, de l'hémisuccinate d'hydrocortisone, de l'EGF, du TGFα, et de l'IGF-1 humains.

Pour compléter un flacon de 10 ml de milieu Upgraded B9, appelé flacon A, nous avons utilisé :
- 12,5 µl d'une solution aqueuse d'insuline humaine à 100 U/ml. Cette solution était présentée sous la forme d'un flacon de 10 ml, appelé ici flacon B. Les tests de sécurité (virus et prions) avaient été préalablement effectués.
- un flacon de lyophilisat d'une capacité de 1,2 ml, appelé ici flacon C, contenant l'hémisuccinate d'hydrocortisone (3,5µg) et les facteurs de croissance et de différenciation humains recombinants EGF (500 ng), TGFα (250 ng), et IGF-1 (125ng). Pour la préparation de ce flacon, l'hémisuccinate d'hydrocortisone (1,4 mg), l'EGF (200 µg), le TGFα (100 µg), et l'IGF-1 (50µg) ont été dissous dans 400 ml d'une solution aqueuse d'albumine humaine sécurisée (1 g/L). Cette solution a été passée sur filtre de 0,2 µm, puis répartie dans 393 flacons de 5 ml, à raison de 1 ml par flacon. Ces derniers ont été lyophilisés. Toutes ces opérations ont été effectuées en milieu stérile.

## Revendications

1. Compositions pour la culture *in vitro* de follicules en cours de développement en vue de la maturation des ovocytes contenus dans lesdits follicules, ou pour le développement d'embryons, éventuellement après décongélation des follicules, ou embryons, **caractérisées en ce qu'**elles comprennent au moins deux facteurs de croissance en association avec au moins un composé de la famille des corticoïdes impliqués dans la production énergétique chez les mammifères, et, le cas échéant, avec au moins un coenzyme clé du métabolisme énergétique, tel que le NAD/NADH et le NADP/NADPH, lesdits facteurs de croissance étant choisis parmi :
- le facteur de croissance hépatique, encore désigné HGF,
- le facteur de croissance de transformation α, encore désigné TGFα,
- le facteur de stimulation de colonies de granulocytes et de macrophages, encore désigné GM-CSF,
- le facteur épidermique de croissance, encore désigné EGF et/ou HB-EGF,
- les facteurs de croissance et de différenciation, encore désignés GDF, tels que le GDF-9,
- les facteurs de croissance semblables-à-l'insuline, encore désignés IGF, tels que l'IGF-1 et/ou l'IGF-2.

2. Composition selon la revendication 1, **caractérisée en ce qu'**elle contient au moins trois facteurs de croissance, ou tous les facteurs listés dans la revendication 1.

3. Composition selon la revendication 1 ou 2, **caractérisée en ce que** les concentrations en facteurs de croissance sont nanomolaires.

4. Composition selon l'une des revendications 1 à 3, **caractérisée en ce que** les concentrations en facteurs de croissance sont comprises entre environ 0,25 µg/L et environ 60 µg/L, notamment entre environ 0,5 µg/L et environ 50 µg/L.

5. Composition selon l'une des revendications 1 à 4, **caractérisée en ce que** le composé de la famille des corticoïdes impliqués dans la production énergétique chez les mammifères, est un composé de la famille des glucocorticoïdes, tel que l'hydrocortisone ou un dérivé de la même famille, sous forme hydrosoluble.

6. Composition selon l'une des revendications 1 à 5, **caractérisée en ce qu'**elle comprend de l'hydrocortisone sous forme de sel tel qu'un hémisuccinate.

7. Composition selon l'une des revendications 1 à 6, **caractérisée en ce qu'**elle se présente sous forme de lyophilisat.

8. Lyophilisats définis dans la revendication 7, contenant :
- au moins deux facteurs de croissance définis dans l'une des revendications 1 à 4, ou la totalité de ces facteurs,
- un composé de la famille des corticoïdes définis dans la revendication 5 ou 6, tel que l'hémisuccinate d'hydrocortisone,
- et, le cas échéant, du NAD/NADH et du NADP/NADPH.

9. Application des compositions ou lyophilisats selon l'une des revendications 1 à 8, en tant qu'adjuvants effecteurs pour la préparation de milieux de culture *in vitro* de follicules en cours de développement en vue de la maturation des ovocytes contenus dans lesdits follicules, ou pour le développement d'embryons, éventuellement après décongélation des follicules, ou embryons, lesdits milieux de culture *in vitro* comprenant une composition selon l'une des revendications 1 à 6, en association avec les éléments utilisés classiquement dans le cadre de la culture des follicules, ou embryons, lesdits éléments étant choisis notamment parmi la sérum albumine humaine, ou bovine, le cas échéant recombinantes, et/ou les sels minéraux, et/ou les molécules énergétiques telles que le glucose, le pyruvate, et le lactate, et/ou les aminoacides pour la biosynthèse des protéines, et/ou les bases puriques et pyrimidiques pour la biosynthèse des acides nucléiques, et/ou des phospholipides ou du cholestérol pour la formation des membranes cellulaires, et/ou des vitamines, telles que des vitamines du groupe B et/ou de la vitamine C.

10. Procédé de préparation de milieux de culture *in vitro* définis dans l'une des revendications 1 à 7, **caractérisé en ce qu'**il comprend une étape de mélange d'une composition selon l'une des revendications 1 à 6, le cas échéant après dissolution dans un volume approprié d'une composition susmentionnée sous forme de lyophilisat selon les revendications 7 ou 8, avec une solution contenant les éléments utilisés classiquement dans le cadre de la culture des follicules, ou embryons, lesdits éléments étant tels que définis dans la revendication 9.

11. Milieux de culture *in vitro* de follicules en cours de développement en vue de la maturation des ovocytes contenus dans lesdits follicules, ou pour le développement d'embryons, éventuellement après décongélation des follicules, ou embryons, lesdits milieux étant tels qu'obtenus par mise en oeuvre d'un procédé selon la revendication 10, et étant **caractérisés en ce qu'**ils comprennent une composition selon l'une des revendications 1 à 6, en association avec les éléments utilisés classiquement dans le cadre de la culture des follicules, ou embryons, lesdits éléments étant tels que définis dans la revendication 9.

12. Procédé *in vitro* de maturation des follicules de mammifères en cours de développement en vue de la maturation des ovocytes contenus dans lesdits follicules, et plus particulièrement de follicules humains, le cas échéant après décongélation de follicules préalablement congelés, **caractérisé en ce qu'**il comprend une étape de mise en culture *in vitro* de follicules prélevés chez la femelle, et plus particulièrement chez la femme, dans un milieu de culture selon la revendication 11, avantageusement pendant environ 3 jours à environ 6 jours.

13. Kit pour la préparation extemporanée d'un milieu selon la revendication 11, notamment dans le cadre de la mise en oeuvre d'un procédé défini dans la revendication 12, **caractérisé en ce qu'**il comprend :
- une composition sous forme de lyophilisat selon les revendications 7 ou 8,
- et, le cas échéant, une solution aqueuse contenant les éléments utilisés classiquement dans le cadre de la culture des follicules, ou embryons, tels que définis dans la revendication 9.

## Claims

1. Compositions for the *in vitro* culture of follicles in the course of development for the maturation of ovocytes contained in said follicles, or for the development of embryos, if desired after thawing of the follicles, or embryos, **characterized in that** they comprise at least two growth factors in association with at least one compound of the family of corticoids implied in energy production in mammals, and, as the case may be, with at least one key co-enzyme of the energy metabolism, such as NAD/NADH and NADP/NADPH, said two growth factors being selected from:
- the hepatocyte growth factor, also called HGF,
- the transforming growth factor α, also called TGFα,
- the granulocyte-macrophage colony stimulating factor, also called GM-CSF,
- the epidermal growth factor, also called EGF and/or HB-EGF,
- the growth-differentiation factors, also called GDF, such as GDF-9,
- the insulin-like growth factors, also called IGF, such as IGF-1 and/or IGF-2.

2. Composition according to claim 1, **characterized in that** it contains at least three growth factors, or all the factors listed in claim 1.

3. Composition according to one of claims 1 or 2, **characterized in that** the concentrations of growth factors are nanomolar.

4. Composition according to one of claims 1 to 3, **characterized in that** the concentrations of growth factors are comprised between about 0.25 µg/L and about 60 µg/L, particularly between about 0.5 µg/L and about 50 µg/L.

5. Composition according to one of claims 1 to 4, **characterized in that** the compound of the family of corticoids implicated in the energy production in mammals, is a compound of the family of glucocorticoids, such as hydrocortisone, or of a derivative of the same family, in water soluble form.

6. Composition according to one of claims 1 to 5, **characterized in that** it comprises hydrocortisone in the form of a salt such as a hemisuccinate.

7. Composition according to one of claims 1 to 6, **characterized in that** it is present in the form of a lyophilizate.

8. Lyophilizates defined in claim 7, containing:
- at least two growth factors defined in one of claims 1 to 4, or all of them,
- a compound of the family of corticoids defined in claim 5 or 6, such as hydrocortisone hemisuccinate,
- and, as the case may be, NAD/NADH and NADP/NADPH.

9. The use of compositions or lyophilizates according to one of claims 1 to 8, as adjuvants effective for the preparation of *in vitro* culture media for follicles in the course of development for the maturation of the ovocytes contained in said follicles, or for the development of embryos, eventually after thawing the follicles, or embryos, said *in vitro* culture media comprising a composition according to one of claims 1 to 6, in association with elements conventionally used in the field of culturing follicles, or embryos, said elements being selected particularly from human albumin serum, or bovine, as the case may be recombinant, and/or mineral salts, and/or energetic molecules such as glucose, pyruvate and lactate and/or amino acids for the biosynthesis of proteins, and/or purine and pyrimidine bases for the biosynthesis of nucleic acids, and/or phospholipids or cholesterol for the formation of cellular membranes, and/or vitamins, such as vitamins of the B group and/or vitamin C.

10. Process for the production of *in vitro* culture media defined in one of claims 1 to 7, **characterized in that** it comprises a step of mixing a composition according to one of claims 1 to 6, as the case may be after dissolution in a suitable volume of a composition mentioned above in the form of a lyophilizate according to claims 7 or 8, with a solution containing elements conventionally used in the field of culturing follicles, or embryos, said elements being as defined in claim 9.

11. *In vitro* culture media for follicles in the course of development for the maturation of ovocytes contained in said follicles, or for the development of embryos, eventually after thawing the follicles, or embryos, said media being as obtained by practicing a process according to claim 10, and being **characterized in that** they comprise a composition according to one of claims 1 to 6, in association with elements conventionally used in the field of culturing follicles, or embryos, said elements being as defined in claim 9.

12. *In vitro* process for the maturation of mammal follicles in the course of development for the maturation of the ovocytes contained in said follicles, and more particularly of human follicles, as the case may be after thawing previously frozen follicles, **characterized in that** it comprises the step of *in vitro* culture of follicles from the female, and more particularly from women, in a culture medium according to claim 11, preferably for about 3 days to about 6 days.

13. Kit for the extemporaneous preparation of a medium according to claim 11, particularly in the scope of practicing a process defined in claim 12, **characterized in that** it comprises:
- a composition in the form of a lyophilizate according to claims 7 or 8,
- and, as the case may be, an aqueous solution containing elements conventionally used in a field of culturing follicles, or embryos, as defined in claim 9.

## Patentansprüche

1. Zusammensetzungen für die *In-vitro*-Kultur von Follikeln während der Entwicklung im Hinblick auf die Reifung der Oozyten, die in den Follikeln enthalten sind, oder zur Entwicklung von Embryonen, eventuell nach dem Auftauen der Follikel oder Embryonen, **dadurch gekennzeichnet, dass** sie mindestens zwei Wachstumsfaktoren umfassen, in Assoziation mit mindestens einer Verbindung der Familie der Corticoide, die an der Energieproduktion beim Säuger beteiligt sind, und gegebenenfalls mit mindestens einem Schlüsselcoenzym des Energiestoffwechsels, wie etwa NAD/NADH und NADP/NADPH, wobei die zwei Wachstumsfaktoren ausgewählt sind aus:
- dem Hepatozyten wachstumsfaktor, auch als HGF bezeichnet,
- dem transformierenden Wachstumsfaktor α, auch als TGFα bezeichnet,
- dem Granulocyte-Macrophage-Colony-stimulating-Faktor, auch als GM-CSF bezeichnet,
- dem epidermalen Wachstumsfaktor, auch als EGF und/oder HB-EGF bezeichnet,
- den Wachstums- und Differenzierungsfaktoren, auch als GDF bezeichnet, wie etwa GDF-9,
- den insulinähnlichen Wachstumsfaktoren, auch als IGF bezeichnet, wie etwa IGF-1 und/oder IGF-2.

2. Zusammensetzung nach Anspruch 1, **dadurch gekennzeichnet, dass** sie mindestens drei Wachstumsfaktoren oder alle Faktoren, die in Anspruch 1 aufgelistet sind, enthält.

3. Zusammensetzung nach Anspruch 1 oder 2, **dadurch gekennzeichnet, dass** die Konzentrationen der Wachstumsfaktoren nanomolar sind.

4. Zusammensetzung nach einem der Ansprüche 1 bis 3, **dadurch gekennzeichnet, dass** die Konzentrationen der Wachstumsfaktoren im Bereich zwischen etwa 0,25 µg/L und etwa 60 µg/L, insbesondere zwischen etwa 0,5 µg/L und etwa 50 µg/L liegen.

5. Zusammensetzung nach einem der Ansprüche 1 bis 4, **dadurch gekennzeichnet, dass** die Verbindung der Familie der Corticoide, die an der Energieproduktion beim Säuger beteiligt sind, eine Verbindung der Familie der Glucocorticoide ist, wie etwa Hydrocortison oder ein Derivat der gleichen Familie in wasserlöslicher Form.

6. Zusammensetzung nach einem der Ansprüche 1 bis 5, **dadurch gekennzeichnet, dass** sie Hydrocortison in Form von Salz, wie etwa einem Hemisuccinat, umfasst.

7. Zusammensetzung nach einem der Ansprüche 1 bis 6, **dadurch gekennzeichnet, dass** sie die Form eines Lyophilisats aufweist.

8. Lyophilisate, definiert in Anspruch 7, enthaltend:
- mindestens zwei Wachstumsfaktoren, definiert in einem der Ansprüche 1 bis 4, oder die Gesamtheit dieser Faktoren,
- eine Verbindung der Familie der Corticoide, definiert in Anspruch 5 oder 6, wie etwa Hydrocortison-Hemisuccinat,
- und gegebenenfalls NAD/NADH und NADP/NADPH.

9. Anwendung der Zusammensetzungen oder Lyophilisate nach einem der Ansprüche 1 bis 8 als Adjuvanzien, die Effektoren für die Herstellung von *In-vitro-*Kulturmedien für Follikel während der Entwicklung im Hinblick auf die Reifung der Oozyten, die in den Follikeln enthalten sind, oder für die Herstellung von Embryonen sind, eventuell nach dem Auftauen der Follikel oder Embryonen, wobei die *In-vitro-*Kulturmedien eine Zusammensetzung nach einem der Ansprüche 1 bis 6 umfassen, in Assoziation mit den Elementen, die herkömmlich im Rahmen der Follikel - oder Embryonenkultur verwendet werden, wobei die Elemente i nsbesondere a usgewählt s ind a us gegebenenfalls rekombinantem, humanem oder bovinem Serumalbumin und/oder Mineralsalzen und/oder Energiemolekülen, wie etwa Glucose, Pyruvat und Lactat und/oder Aminosäuren für die Biosynthese der Proteine und/oder Purin- und Pyrimidinbasen für die Biosynthese der Nukleinsäuren, und/oder Phospholipiden oder Cholesterol zur Bildung der Zellmembranen und/oder Vitaminen, wie etwa Vitaminen der Gruppe B und/oder Vitamin C.

10. Verfahren zur Herstellung von *In-vitro*-Kulturmedien, definiert in einem der Ansprüche 1 bis 7, **dadurch gekennzeichnet, dass** es einen Schritt zum Mischen einer Zusammensetzung nach einem der Ansprüche 1 bis 6 ümfasst, gegebenenfalls nach Auflösen in einem geeigneten Volumen einer oben genannten Zusammensetzung in Form eines Lyophilisats nach den Ansprüchen 7 oder 8 mit einer Lösung, die die Elemente enthält, die herkömmlich im Rahmen der Follikel- oder Embryonenkultur verwendet werden, wobei die Elemente so sind, wie in Anspruch 9 definiert.

11. *In-vitro*-Kulturmedien für Follikel während der Entwicklung im Hinblick auf die Reifung der Oozyten, die in den Follikeln enthalten sind, oder zur Entwicklung von Embryonen, eventuell nach dem Auftauen der Follikel oder Embryonen, wobei die Medien so sind, wie durch Durchführung eines Verfahrens nach Anspruch 10 erhalten, und **dadurch gekennzeichnet, dass** sie eine Zusammensetzung nach einem der Ansprüche 1 bis 6 in Assoziation mit den Elementen umfassen, die herkömmlich im Rahmen der *In-vitro*-Fertilisation oder der Follikel- oder Embryonenkultur verwendet werden, wobei die Elemente so sind, wie in Anspruch 9 definiert.

12. *In-vitro*-verfahren zur Reifung der Follikel von Säugern während der Entwicklung im Hinblick auf die Reifung der Oozyten, die in den Follikeln enthalten sind, und ganz besonders der humanen Follikel, gegebenenfalls nach dem Auftauen von zuvor eingefrorenen Follikeln, **dadurch gekennzeichnet, dass** es einen Schritt zur *In-vitro*-Kultivierung von Follikeln, die bei dem Weibchen und insbesondere bei der Frau entnommen wurden, in einem Kulturmedium nach Anspruch 11, vorteilhafterweise für etwa 3 Tage bis etwa 6 Tage, umfasst.

13. Kit zur Herstellung eines Mediums nach Anspruch 11 unmittelbar vor der Verwendung insbesondere im Rahmen der Durchführung eines in Anspruch 12 definierten Verfahrens, **dadurch gekennzeichnet, dass** es umfasst:
- eine Zusammensetzung in Form eines Lyophilisats nach den Ansprüchen 7 oder 8,
- und gegebenenfalls eine wässrige Lösung, die die Elemente enthält, die herkömmlich im Rahmen der ' Follikel- oder Embryonenkultur verwendet werden, wie in Anspruch 9 definiert.
